(19) Europäisches Patentamt
European Patent Office
Office européen des brevets

(11) Publication number: 0 422 402 A2

(12) **EUROPEAN PATENT APPLICATION**

(21) Application number: 90117594.3

(22) Date of filing: 12.09.90

(51) Int. Cl.5: C07D 251/18, C08K 5/3492

(30) Priority: 13.10.89 US 421044

(43) Date of publication of application: 17.04.91 Bulletin 91/16

(84) Designated Contracting States: AT BE CH DE DK ES FR GB GR IT LI LU NL SE

(71) Applicant: AMERICAN CYANAMID COMPANY
1937 West Main Street P.O. Box 60
Stamford Connecticut 06904-0060(US)

(72) Inventor: Bright, John Harvey
8 Bayne Court
Norwalk, Connecticut 06851(US)
Inventor: Wu, Kuang-Jong
15 Stonewall Lane
Shelton, Connecticut 06484(US)
Inventor: Brogan, John Colin
21 Nelson Street
Stamford, Connecticut 06902(US)

(74) Representative: Wächtershäuser, Günter, Dr.
Tal 29
W-8000 München 2(DE)

(54) **Thermosetting coating compositions with etherified methylolated aliphatic guanamines.**

(57) Disclosed is a method of preparing substantially fully methylolated substantially fully etherified, and substantially monomeric N,N,N′,N′-tetraalkoxymethyl cyclohexanecarboguanamine crosslinking agents. These crosslinking agents have viscosities of less than 1000 centipoises and are useful in curable compositions in combination with polyfunctional materials such as acrylic and polyester resins.

EP 0 422 402 A2

# THERMOSETTING COATING COMPOSITIONS WITH ETHERIFIED METHYLOLATED ALIPHATIC GUANAMINES

## FIELD OF THE INVENTION

This invention relates to curable compositions comprising etherified aliphatic methylolated guanamines and thermosetting coatings thereof.

## BACKGROUND OF THE INVENTION

Thermosetting coating compositions of etherified melamine-formaldehyde resins are well known in the art. Due to their many advantages, they are used extensively in coatings, in particular in automotive top coats. Despite their widespread use, etherified melamine-formaldehyde resins suffer from poor storage stability and short pot life. Also, the thermosetting products thereof are often very rigid, even brittle due to the high functionality of the melamine aminoplast crosslinking agent.

Benzoguanamine-based resins of similar type have been used in the place of melamine-based systems to minimize the disadvantages of the melamine resins, but they have introduced new disadvantages of their own. For example, even though etherified benzoguanamine-formaldehyde resins have superior storage stability and flexibility in coatings applications, they have inferior light stability and discolor rapidly when exposed to ultraviolet light. This aspect has limited their use to coating applications with only a modest demand for light stability.

In contrast to the systems described above, etherified aliphatic guanamine-formaldehyde resins have received little attention as alternatives to the melamine and benzoguanamine aminoplast crosslinkers.

U.S. Patent 3,663,389 and also U.S. Patents 3,894,993 and 3,945,961 disclose coating compositions with substantially fully etherified, substantially fully methylolated aliphatic guanamines, whereby the aliphatic substituent group possesses 1-8 carbon atoms. The compositions described in these patents, however, are usable only in electrodeposition coatings, and not in general thermosetting applications.

U.S. Patent No. 4,221,683 describes low temperature curing coating compositions comprising etherified amino-formaldehyde resins with a brief mention of several aliphatic and aromatic guanamines.

European Patent Application 292,306 discloses etherified methylolaminotriazine condensates with cyclohexyl and norbornyl substituents, and a method for their preparation and European Patent Application 303,441 discloses coatings compositions thereof. It is taught therein that cured thermosetting compositions comprising said condensates have superior flexibility, weatheribility, gloss, and corrosion resistance. These systems, however, are believed to be highly oligomeric resulting in very viscous compositions not well suited for high solids coatings applications. Furthermore, the highly oligomeric condensates of these disclosures have been found to be very sluggish in their cure response. While some moderation of the crosslinking rates may be desirable to improve storage stability and pot-life, sluggish cure response is generally very undesirable. Slow curing systems require high levels of a cure catalyst to overcome the sluggishness in their cure response. It is well known in the coatings industry that high level usage of cure catalysts ultimately leads to poor corrosion and humidity resistance, and poor overall physical properties of the coated films.

It is the object of this invention to provide an acid-curable, high solids and low viscosity thermosetting coating compositions which have the proper balance between storage stability and rapid cure response.

It is another object of this invention to provide coating compositions comprising monomeric form of etherified methylolated aliphatic guanamine crosslinkers which cure more rapidly than their oligomeric analog and impart a balance of properties which are necessary in high performance applications.

It is another object of this invention to provide coatings by curing the above compositions to produce coatings which have superior flexibility and light stability.

It is yet another object of this invention to provide a process for the preparation of the substantially fully methylolated and substantially fully etherified substantially monomeric aliphatic guanamine crosslinking agents of this invention.

## SUMMARY OF THE INVENTION

This invention provides a method for the preparation of substantially fully methylolated and substantially fully etherified, substantially monomeric cyclohexanecarboguanamine crosslinking agents. This invention is also the novel cyclohexanecarboguanamine crosslinking agents produced by the process of the invention. This invention also provides acid-curable, high solids, low viscosity compositions comprising:

(i) a polyfunctional material capable of reacting with alkoxymethyl group containing crosslinking agents

(ii) a crosslinkingly effective amount of an N,N,N′,N′-tetraalkoxymethyl cyclohexanecarboguanamine crosslinking agent (Formula I).

(iii) a curing catalyst.

This invention also provides a method of producing flexible and light stable coatings and coated articles obtained by curing the acid-curable compositions of this invention.

## DETAILED DESCRIPTION OF THE INVENTION

### Preparation of the Crosslinkers

The novel substantially monomeric crosslinking agents of this invention are produced by the following sequential steps, removing water or distilling water with an alcohol after each step:

(a) methylolating cyclohexanecarboguanamine represented by the Formula (II):

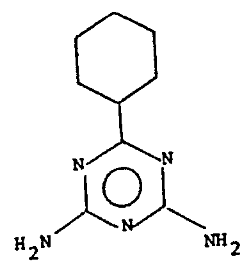

(II)

with formaldehyde at a pH range of 7-10 and a temperature range of 0-150°C, to obtain substantially fully methylolated substantially monomeric N,N,N′,N′-tetramethylol cyclohexanecarboguanamine represented by the Formula (III):

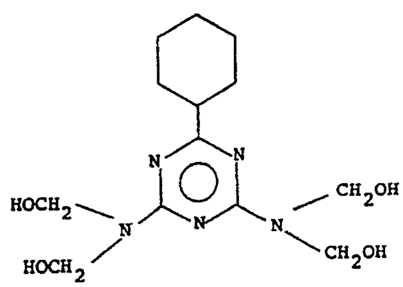

(III)

(b) Partially etherifying the substantially fully methylolated, substantially monomeric N,N,N′,N′-tetramethylol cyclohexanecarboguanamine compound (III), which is the product of step (a), with an alcohol of 1-4 carbon atoms, or a mixture thereof, at a pH range of 0-3 and a temperature range of 0-50°C.

(c) Further etherifying the partially etherified reaction product of step (b) at a pH range of 0-3 and a temperature range of 0-50°C with an alcohol of 1-4 carbon atoms, or a mixture thereof.

(d) Repeating step (c) until no additional water can be removed.

Common organic solvents are generally suitable for the preparation of the crosslinkers of this invention. These include water, alcohols, ketones, ethers, esters, aromatic and aliphatic compounds and their halides, and mixtures thereof. Alcohols and mixtures of alcohols and water are particularly useful in the preparation of the crosslinking agents of this invention because alcohols can be used both as solvent and etherification

agents.

It is critical, however, to carry out the sequential steps (b), (c), and (d) at a pH less than 3 and a temperature below 50°C because at pH values higher than 3 or at temperatures above 50°C significant oligomerization takes place.

The use of methanol or normal butanol as etherification agents for forming the crosslinking agent is preferred.

It is a discovery of this invention that the N,N,N′,N′-tetraalkoxymethyl cyclohexanecarboguanamine crosslinking agents produced as described above have specific curing properties not found in prior art materials. Therefore, without being bound by any theory of operation, it is believed that the crosslinking agents of this invention differ in some manner from those described in the prior art. Nevertheless, the general structure of the crosslinking agents is believed to be substantially fully methylolated and substantially fully etherified, substantially monomeric cyclohexanecarboguanamine crosslinking agents represented by the Formula (I):

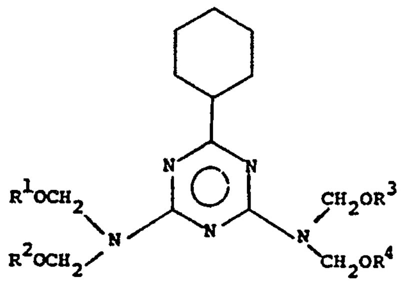

(I)

wherein $R^1$, $R^2$, $R^3$, $R^4$ are the same or different alkyl radicals of 1-4 carbon atoms, inclusive. The N,N,N′,N′-tetraalkoxymethyl cyclohexanecarboguanamine crosslinking agents are not considered to be resinous materials since they are, as individual entities, non-polymeric compounds. They are, however, potential resin-forming compounds when subjected to heat, and particularly when subjected to heat under acidic conditions. As a result of the described resin-forming ability of the substantially monomeric crosslinkers of this invention they may contain low levels of higher, oligomeric structures such as dimers, trimers, and perhaps even tetramers. The presence of small amounts of these oligomeric forms is permissible and it is not necessary to exclude them. The monomeric forms of the crosslinker of the invention have viscosities of less than 1000 centipoise.

Curable Compositions:

The novel low temperature and low acid curable compositions of this invention comprise as essential ingredients:

(i) a polyfunctional material capable of reacting with alkoxymethyl groups containing crosslinking agents.

(ii) a crosslinkingly effective amount of N,N,N′,N′-tetraalkoxymethyl cyclohexanecarboguanamine crosslinking agents.

(iii) an acid cure catalyst.

The ingredients (i) and (ii) in combination constitute the "total resin solids" of the composition.

A. Polyfunctional Alkoxymethyl-Reactive Material

Polyfunctional materials, capable of reacting with polyfunctional alkoxymethyl-containing crosslinking agents constitute one of the three essential components required for the curable compositions of this invention. The other necessary components are an N,N,N′,N′-tetraalkoxymethyl cyclohexanecarboguanamine crosslinking agent, and an acid cure catalyst.

The alkoxymethyl-reactive functional groups are selected from materials containing amino groups, amido groups, carboxy groups, mercapto groups, hydroxy groups, or combinations of any of the aforementioned groups. Alkoxymethyl-reactive materials having a plurality of hydroxy groups are particularly preferred in the practice of the invention.

Suitable polyfunctional hydroxy-containing materials may be acrylic resins containing pendant hydroxy functionalities, or polyester resins with terminal hydroxy groups. They are described in greater detail below.

Acrylic Resin Methoxymethyl-Reactive Material

The hydroxyfunctional acrylic resins used in formulating the curable compositions of this invention preferably have molecular weights in the range from about 1,000 to 50,000 and hydroxyl group equivalent weights of from about 200 to 2,000. An example of a suitable acrylic resin is JONCRYL 500® Acrylic Resin, a product of S.C. Johnson & Son, Inc., of Racine, WI, having the following physical and chemical properties:
Hydroxyl Number (solids) : 140
Equivalent Weight (solids): 400
Molecular Weight (Mn) : 1,300
Polydispersity (Mw/Mn) : 1.7

Commercially available acrylic resins are generally suitable for use in preparing the curable compositions of the invention. Selection of the optimum acrylic resin will depend on the user's desire to impart to the final cured product particular physical properties such as hardness, softness, flexure, chemical or solvent resistance, and the like.

Polyester Resin Methoxymethyl-Reactive Material

The hydroxyfunctional polyester resins used in formulating the curable compositions of this invention preferably have molecular weights in the range of from about 1,000 to 50,000 and hydroxyl group equivalent weights of from about 200 to 2,000. An example of a suitable polyester resin is CYPLEX 1531, Polyester Resin, a product of American Cyanamid Company, Wayne, NJ, having the following physical and chemical properties:
Hydroxyl Number (solids) : 30
Equivalent Weights (solids) : 1,870
Molecular Weight : 4,000

A wide variety of commercial polyester resins may be used as the methoxymethyl-reactive ingredient of the invention to impart specifically desired physical properties to the cured materials.

Other Methoxymethyl-Reactive Systems

Other polyfunctional hydroxy compounds which are useful in the practice of the invention are diols, triols, tetraols, and higher polyhydroxy alcohols. Illustrative polyhydroxy alcohols are ethylene glycol, propylene glycol, butane diol, hexane diol, pentaerythritol, dipentaerythritol, tripentaerythritol, glycerol, trimethylolethane, trimethylolpropane, and the like. These polyols can also be used to totally or partially replace the acrylic resin or polyester resin methoxymethyl-reactive ingredient of the curable compositions of the invention.

Polyfunctional amides or urethanes are also suitable as alkoxymethyl-reactive components, however, to be effective as alkoxymethyl-reactive components, the polyfunctional amides and urethanes must contain alkoxymethyl-reactive primary $NH_2$-functional groups. Similarly, polyfunctional mercaptans and polyfunctional carboxylic acids are useful as alkoxymethyl-reactive components in the curable compositions of this invention.

B. Crosslinking Agents

The crosslinkers usable in curable compositions of this invention are the novel, substantially fully methylolated, substantially fully etherified, substantially monomeric N,N,N′,N′-tetraalkoxymethyl cyclohexanecarboguanamines represented by Formula (I):

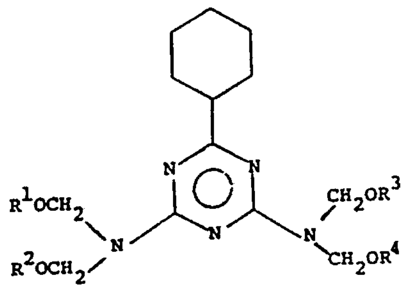

(I)

wherein $R^1$, $R^2$, $R^3$, $R^4$ are the same or different alkyl radicals of 1-4 carbon atoms, inclusive.

### C. Acid Cure Catalyst

The third essential ingredient of the curable compositions of this invention is an acid cure catalyst. The most suitable acid cure catalysts are organic sulfonic acids and include methanesulfonic acid, para-toluene sulfonic acid, benzenesulfonic acid, mesitylenesulfonic acid, naphthalenesulfonic acid, dodecylbenzenesulfonic acid and the like. Other acids such as phosphonic acids and carboxylic acids may also be used. Examples of the phosphonic acids are dimethylphosphonic acid, and diphenyl phosphonic acid. Examples of the carboxylic acids are acetic acid, formic acid and oxalic acid. The acid cure catalyst ingredient in the curable compositions of this invention is present for the purpose of accelerating cure rate.

### The Ratio and Proportion of Essential Ingredients in the Curable Composition

The polyfunctional alkoxymethyl-reactive material ingredient of the curable compositions of this invention is combined with the N,N,N′,N′-tetraalkoxymethyl cyclohexanecarboguanamine crosslinking agent ingredient of the curable compositions of this invention in proportions ranging between 2.0 to 0.1 equivalents of the polyfunctional alkoxymethyl-reactive material per each equivalent of the N,N,N′,N′-tetraalkoxymethyl cyclohexanecarboguanamine crosslinking agent.

The acid cure catalyst ingredient of the curable compositions of the invention is combined with the remaining essential ingredients and in proportions less than 4.65 millimoles of an acid per each 100 grams of total resin solids and preferably 2.33 or less millimoles of an acid per each 100 gram of total resin solids.

The combining of ingredients of the curable compositions does not require any particular order, but usually the crosslinker is added last to minimize self-reaction.

### Crosslinked Articles and Coatings Formed by Curing the Compositions of this Invention

Curable compositions comprising the essential ingredients are cured by thermal activation of the crosslinking process wherein the polyfunctional alkoxymethyl-reactive functional groups react with the alkoxymethyl functional groups present on the crosslinking agent resulting in crosslinked material. These materials can be in the form of a crosslinked film such as coatings, or can be in the form of a crosslinked article such as molded products.

The heat-cured compositions of this invention may be employed as coatings for wire, appliances, automotive parts, furniture, pipes, machinery, beverage cans, and the like. Suitable surfaces include metals such as steel and aluminum.

The following examples illustrate various embodiments of the invention:

## EXAMPLE 1

This example illustrates the preparation, by the process of this invention, of "monomeric" N,N,N′,N′-tetramethoxymethyl cyclohexanecarboguanamine, a crosslinking agent of this invention.

Step (a):

A mixture of cyclohexanecarboguanamine (II) (193.3 g, 1.0 mole), 44% aqueous formaldehyde (547 g, 8.0 mol), and 50% aqueous sodium hydroxide (0.608 g) having a pH of 8.6-8.8, was heated to 70-79°C for 30 minutes. The volatiles (about 232 g) were removed under reduced pressure (corresponding to 66 to 84 millimeters of mercury). The reaction mixture was then cooled to 36°C.

Step (b):

Methanol (288 g) was added to the product of step (a). Then 69% nitric acid (19.3 g, 14 ml) was also added to lower the pH to about 2.6. After 35 minutes at 36-42°C and pH 2.5-2.6, the pH of the mixture was raised to 10.4 by adding 50% aqueous sodium hydroxide (19.8 g, 13 ml). Then the temperature was raised to 62°C and the volatiles (~340 g) were partially removed under reduced pressure (~ 76 mm of mercury). The reaction mixture was then cooled to 40°C.

Step (c):

Methanol (402 g) was added to the product of step (b). Then 69% nitric acid (20.2 g, 14.5 ml) was also added to lower the pH to about 2.85. After 45 minutes at 40-48°C and pH 2.85, the pH of the mixture was raised to 9.9 by adding 25% aqueous sodium hydroxide (39.0 g, 31.6 ml). Then the temperature was raised to 105°C and the volatiles (342.2 g) were removed under reduced pressure (81.3 mm of mercury). The reaction mixture was then cooled to 26°C.

Step (d):

Methanol (405.3 g) was added to the product of step (c). Then 69% nitric acid (19.6 g, 14.0 ml) was also added to lower the pH to about 2.4. After 45 minutes at 45-49°C and pH 2.4, the pH of the mixture was raised to 9.78 by adding 16% aqueous sodium hydroxide (57.9 g). Then the temperature was raised to 106°C and the volatiles (337 g) were removed under reduced pressure (~ 76 mm mercury).

The suspended salts were removed by filtering the product of step (d) at 70°C under pressure. The filtrate was N,N,N′,N′-tetramethoxymethylcyclohexylcarboguanamine. High Performance Size Exclusion Chromatography (HPSEC) revealed that the product was substantially monomeric (97%) with a small amount (3%) of a dimer. No trimers or tetramers were detected. Other physical properties of the "monomeric" crosslinker of the invention are listed in Table 1, and the method of preparation is summarized in Table 2 as set out below.

Table 1

| Physical Properties of Crosslinkers of Formula I | | | |
|---|---|---|---|
| | Example 1 Monomeric, $R^1, R^2, R^3, R^4$ = Methyl | Example 2 Oligomeric $R^1, R^2, R^3, R^4$ = Methyl | Example 3 Monomeric, $R^1, R^2, R^3, R^4$ = n-Butyl |
| Composition (moles per mole of crosslinker of Formula I) | $F_{3.94}Me_{3.94}$[a] | $F_{3.4}Me_{3.1}$[a] | $F_{4.3}Bu_{3.7}$[a] |
| Composition (%) by HPSEC[b] | | | |
| Monomer | 97 | 70 | 97 |
| Dimer | 3 | 21 | 3 |
| Trimer | 0 | 7 | 0 |
| Tetramer | 0 | 3 | 0 |
| Viscosity | | | |
| (Gardner-Holdt) | R | W | G |
| (Centipoise) | 470 | 1070 | 165 |
| Non-volatiles wt% (45°C for 45 min.) | >99 | 94 | 96 |
| Solvent | none | n-butanol | n-butanol |
| Free Formaldehyde, wt % | <0.1 | 0.26 | <0.1 |
| Relative N-H[c] | 1.5 | 10 | 2 |
| Average MW[d] | 379 | 514 | 558 |

a. F = Formaldehyde; Me = Methanol; Bu = Normal butanol; composition obtained by Nuclear Magnetic Resonance spectroscopy.
b. High Performance Size Exclusion Chromatography.
c. Relative N-H values obtained by Infrared Spectroscopy.
d. Calculated molecular weights based on composition.

Table 2

| Preparation of Monomeric and Oligomeric N,N,N′,N′-tetramethoxymethyl cyclohexanecarboquanamines | | |
|---|---|---|
| Charges Ratios | Monomeric Form[a] | Oligomeric Form[b] |
| (cyclohexanecarboguanamine/Formaldehyde/$CH_3OH$/$CH_3OH$/$CH_3OH$) | 1/8/9/12.5/2.6 | 1/7/8.25/10/6 |
| Step (a) | | |
| Methylolation | | |
| pH | 8.8-8.6 | 9.5-9.7 |
| Temperature | 70-79°C, | 73-60°C, |
| Time | 30 min. | 35 min. |
| Strip | partial | partial |
| Step (b) | | |
| Etherification | | |
| pH | 2.6-2.5 | 2.9-3.5 |
| Temperature | 36-42°C, | 29-28°C, |
| Time | 35 min. | 60 min. |
| strip | partial | partial |
| Step (c) | | |
| Etherification | | |
| pH | 2.85 | 4.2 |
| Temperature | 40-48°C, | 51-53°C, |
| Time | 45 min. | 55 min. |
| strip | 105°C | partial |
| Step (d) | | |
| Etherification | | |
| pH | 2.4 | 3.4 |
| Temperature | 45-48°C, | 71-74°C, |
| Time | 45 min. | 65 min. |
| strip | 106°C | 102°C |

a. Crosslinker of Example 1.
b. Crosslinker of Example 2.

## EXAMPLE 2

This example illustrates the preparation, by a process which is outside the scope of this invention, of "oligomeric" N,N,N′,N′-tetramethoxymethyl cyclohexanecarboguanamine, a crosslinker which is outside the scope of this invention.

The procedures of Example 1 was followed but the pH of step (b), step (c), and step (d) were above pH 3 and the temperature of step (c) and step (d) were above 50°C. A comparison of the experimental conditions between this Example and the novel process of Example 1 is set out in Table 2. At the end of the reaction, the suspended salts were removed by adding n-butanol (30 g) to the product of step (d) of this example and filtering at 95°C under pressure. The filtrate was N,N,N′,N′-tetramethoxymethyl cyclohexanecarboguanamine. High Performance Size Exclusion Chromatography (HPSEC) revealed that the product was only 70% "monomeric", with the remaining 30% being "oligomeric". The product had the following distribution:

Monomeric : 70%
Dimeric : 21%
Trimeric : 7%
Tetrameric: 3%

Other physical properties of the "oligomeric" crosslinker outside the scope of this invention described in Example 2 are listed in Table 1 for the purpose of comparison with a "monomeric" crosslinker of this invention.

## EXAMPLE 3

This example illustrates the preparation, by the method of the invention, of N,N,N′,N′-tetra-n-butoxymethyl cyclohexanecarboguan amine, a crosslinking agent of the invention.

To prepare the n-butoxymethyl-containing crosslinking agent of the invention, the procedure of Example 1 was followed with the exception of using normal butanol in the lace of methanol as the etherification reagent. The pH of the reaction mixture was kept below 3 and the temperature was kept below 50°C during the etherification reaction in steps (b), (c), and (d).

The reaction product was filtered once in a preheated (90°C) filter to give substantially fully methylolated, substantially fully etherified, substantially monomeric N,N,N′,N′-tetra-n-butoxymethyl cyclohexanecarboguanamine crosslinking agent. High Performance Size Exclusion Chromatography revealed that the reaction product was substantially monomeric (97%) with small amounts (3%) of a dimer. No trimers or tetramers were detected. The remaining physical properties of the monomeric crosslinker are listed in Table 1.

## EXAMPLE 4

This example illustrates that the "monomeric" form of N,N,N′,N′-tetramethoxymethyl cyclohexanecarboguanamine, which is a crosslinking agent of the invention, cures much faster than the "oligomeric" form of N,N,N′,N′-tetramethoxymethyl cyclohexanecarboguanamine, which is a crosslinking agent outside the scope of this invention. The curable composition (A) with the "monomeric" form described herein is an example of a curable composition of this invention.

| FORMULATION OF CURABLE COMPOSITIONS | |
|---|---|
| A. COMPOSITION A | |
| Curable Composition with the "Monomeric" Form | |
| Joncryl® 500 Acrylic Resin, 80% Solids,[1] (g) | 87.5 |
| Cycat® 4040 Cure Catalyst[2] (40% para-Toluenesulfonic Acid) (g) | 1.0 |
| "Monomeric" Crosslinker of Example 1 (g) | 30.0 |
| Normal Butanol (g) | 15.0 |
| Xylene (g) | 21.0 |
| Non-Volatiles (%) | 65.0 |
| B. COMPOSITION B | |
| Curable Composition with the "Oligomeric" Form | |
| Joncryl® 500 Acrylic Resin, 80% Solids[1], (g) | 87.5 |
| Cycat® 4040 Cure Catalyst[2], (40% para-Toluenesulfonic Acid) (g) | 1.0 |
| "Oligomeric" Crosslinker of Example 2 | 31.9 |
| Normal Butanol (g) | 15.0 |
| Xylene (g) | 19.1 |
| Non-Volatiles (%) | 65.0 |

[1] A product of S.C. Johnson & Son, Inc., Racine, WI, 53403-5011
[2] A product of American Cyanamid Company, Wayne, NJ, 07470

C. COMPOSITION C

Curable Composition with the "Monomeric" Form at Twice the Level of Catalyst

The formulation of composition A was used with the exception that 2.0% Cycat® 4040 Cure Catalyst was used instead of 1.0%.

D. COMPOSITION D

Curable Composition with the "Oligomeric" Form at Twice the Level of Catalyst

The formulation of Composition B was used with the exception that 2.0% Cycat® Cure Catalyst was used instead of 1.0%.

E. Curing of the Curable Compositions A and B

The curable compositions A and B of this Example were applied on Bonderite 100 Cold Roll Steel (CRS) substrate panels by means of a #46 wire cator applicator and were cured by heating at a cure schedule of 30 minutes at 250°F (121°C). The cured film was evaluated by standard methods widely accepted by the coatings industry. The physical properties of the cured films obtained by curing composition A, a curable composition of the invention prepared with a "monomeric" crosslinker of the invention, and composition B, a curable composition outside the scope of the invention prepared by an "oligomeric" crosslinker outside the scope of this invention, are listed in Table 3.

Compositions "C" and "D" were also cured in the above manner. Both of these compositions were outside the scope of the invention.

Table 3

| Comparison of the Cure Response of Monomeric and Oligomeric Forms of Crosslinkers of Formula I with Joncryl 500 Acrylic Resin in Unpigmented Formulations | | |
|---|---|---|
| Acrylic/crosslinker ratio | 70/30 | |
| Substrate | Zinc Phosphated CRS[b] | |
| Bake Schedule | 121°C for 30 minutes | |
| Crosslinker of Formula (I) | | |
| $R^1$, $R^2$, $R^3$, $R^4$, = Methyl | Monomeric Form | Oligomeric Form |
| Composition | A | B |
| 0.4% PTSA[a] on Total Resin Solids | | |
| Film thickness, milm/mm<br>Tukon hardness<br>MEK rubs (to mar)<br>(to remove) | 1.6/0.04<br>9.0<br>200+ | 1.6/0.04<br>5.2<br>5<br>125 |
| Composition | C | D |
| 0.8% PTSA[a] on Total Resin Solids | | |
| Film thickness, mils/mm<br>Tukon hardness<br>MEK rubs (to mar)<br>(to remove) | 1.6/0.04<br>9.9<br>200+ | 1.6/0.04<br>9.3<br>200<br>200+ |

[a]. para-Toluene Sulfonic Acid available from American Cyanamid Company as a 40% solution in xylene under the trade name Cycat® 4040 Cure Catalyst; 0.4 weight percent PTSA = 2.33 millimoles PTSA per 100 grams of total resin solids.
[b]. Cold Roll Steel

EXAMPLE 5

This example illustrates that the "monomeric" form of N,N,N′,N′-tetramethoxymethyl cyclohexanecarboguanamine, which is a crosslinking agent of the invention, cures faster than the "oligomeric" form of N,N,N′,N′-tetramethoxymethyl cyclohexanecarboguanamine, which is a crosslinking agent outside the scope of this invention. The curable composition with the "monomeric" form described herein is an example of a curable composition of this invention.

| FORMULATION OF CURABLE COMPOSITIONS | |
|---|---|
| A. COMPOSITION A | |
| Curable Compositions with the "Oligomeric" Form | |
| Cyplex® 1531 Polyester Resin, 60% Solids(1) (g) | 143.2 |
| Cycat® 4040 Cure Catalyst(1), (40% para-Toluenesulfonic Acid) (g) | 0.75 |
| "Oligomeric" Crosslinker of Example 2 (g) | 15.0 |
| $TiO_2$ Pigment (g) | 80.0 |
| Normal Butanol (g) | 15.0 |
| Solvesso 150 Solvent (g) | 15.0 |
| L 7502 Flow Control Agent(2) (g) | 0.2 |
| B. COMPOSITION B | |
| Curable Compositions with the "Monomeric" Form | |
| Cyplex® 1531 Polyester Resin, 60% Solids(1) (g) | 141.7 |
| Cycat® 4040 Cure Catalyst(1), (40% para-Toluenesulfonic Acid) (g) | 0.75 |
| "Monomeric" Crosslinker of Example 1 (g) | 15.0 |
| $TiO_2$ Pigment (g) | 80.0 |
| Normal Butanol (g) | 15.0 |
| Solvesso 150 Solvent (g) | 15.0 |
| L 7502 Flow Control Agent(2) (g) | 0.2 |

(1) A product of American Cyanamid Company
(2) A product of Union Carbide Corporation

F. Curing of the Curable Compositions A and B

The curable compositions A and B were applied on Borderite 1000 Cold Roll Steel (CRS) ferric phosphate treated substrate panels by means of a #34 wire cator applicator. After 5 minutes flash-off time, the panels were cured by heating at several cure schedules depicted in Table 4. The cured film was evaluated by standard methods widely accepted by coatings industry. PMT in Table 4 refers to "Peak Metal Temperature". T-Bend measurements were carried out according to Standard Testing Procedure #500. The physical properties of the cured films are listed in Table 4.

## Table 4

**Polyester Coil Coating Compositions of Crosslinker of Formula I With CYPLEX 1531 POLYESTER**

**Comparison of Monomeric and Oligomeric Crosslinkers**

| | |
|---|---|
| Cyplex 1531/CLA Ratio | 85/15 |
| PTSA, % on total resin solids | 0.3 |
| Titanium dioxide/total resin solids | 0.8 |
| Bake temperature, °C | 300 |
| Substrate | Zinc phosphated CRS |
| Film thickness, mils/mm | 1.0/0.025 |

| Bake Time, sec | 40 | | 45 | | 50 | | 55 | | 60 | | 65 | | 70 | |
|---|---|---|---|---|---|---|---|---|---|---|---|---|---|---|
| PMT, °C | 204 | | 210 | | 216 | | 224 | | 224-232 | | 232 | | 238 | |
| Formula (I), $R^1$, $R^2$, $R^3$, $R^4$ = Methyl | Oligomeric | Monomeric | Oligomeric | Monomeric | Oligomeric | Monomeric | Oligomeric | Monomeric | Oligomeric | Monomeric | Oligomeric | Monomeric | Oligomeric | Monomeric |
| Knoop Hardness | 10.4 | 7.5 | 13.3 | 10.0 | 14.0 | 12.9 | 14.3 | 13.7 | 14.3 | 14.8 | 14.2 | 14.3 | 12.6 | 13.6 |
| T-Bend | T-1 | T-0 | T-1 | T-1 | T-2 | T-2 | T-2 | T-2 | T-3 | T-3 | T-3 | T-3 | T-3 | T-3 |
| MEK (to attack) | 20 | 20 | 50 | 40 | 100 | 75 | 100 | 100 | 100 | 150 | 100 | 150 | 50 | 100 |
| (to remove) | 200+ | 200+ | 200+ | 200+ | 200+ | 200+ | 200+ | 200+ | 200+ | 200+ | 200+ | 200+ | 200+ | 200+ |

It is concluded from the results summarized in Table 4 that a curable composition of this invention which contains the "monomeric" form of N,N,N',N'-tetramethoxymethyl cyclohexaneguanamine crosslinker

(a) cures faster and therefore more completely than the composition which contains the "oligomeric" form at peak metal temperatures between 224°C to 232°C and 60 to 65 seconds.

(b) has a better overbake tolerance at 238°C temperatures and 70 seconds cure time.

Although the present invention has been described with reference to certain preferred embodiments, it is apparent that modifications and changes may be made therein by those skilled in the art without departing from the scope of this invention as defined by the appended claims.

## Claims

1. A method of making N,N,N',N'-tetraalkoxymethyl cyclohexanecarboguanamines represented by the Formula (I):

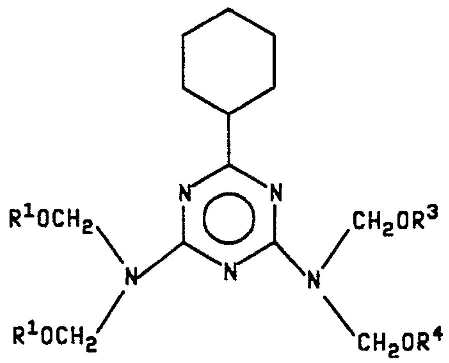

(I)

wherein $R^1$, $R^2$, $R^3$, $R^4$ are the same or different alkyl radicals of 1-4 carbon atoms, inclusive comprising the following sequential steps:

(a) methylolating cyclohexanecarboguanamine;

(b) partially etherifying the reaction product of step (a) at a pH less than 3 and at a temperature below 50°C;

(c) etherifying the reaction product of step (b) further at a pH less than 3 and at a temperature below 50°C.

(d) repeating step (c) as necessary until no additional water can be removed.

2. The process of Claim 1 wherein the operating temperature of step (b) is from 0°C to 50°C and the pH of step (b) is from 1 to 3.

3. The process of Claim 1 wherein the operating temperature of step (c) is from 0°C to 50°C and the pH of step (c) is from 1 to 3.

4. The process of Claim 1 wherein the etherification of steps (b) and (c) is carried out with methanol or butanol.

5. A substantially monomeric N,N,N',N'- tetraalkoxymethyl cyclohexanecarboguanamine having a viscosity of 1000 or less centipoise based on 99 wt % solids content represented by the Formula (I)

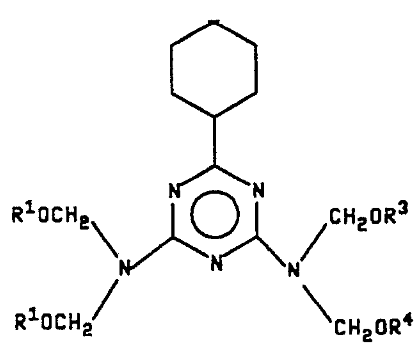

(I)

wherein $R^1$, $R^2$, $R^3$, $R^4$ are the same or different alkyl radicals of 1-4 carbon atoms, inclusive.

6. The guanamine of Claim 5 wherein $R^1$, $R^2$, $R^3$, $R^4$ are methyl, having a viscosity of less then 500 centipoise.

7. The guanamine of Claim 5 wherein $R^1$, $R^2$, $R^3$, $R^4$ are butyl, having a viscosity of less than 500 centipoises.

8. A fast curing composition comprising as essential ingredients:

(i) a polyfunctional material capable of reacting with alkoxymethyl group containing crosslinking agents.

(ii) a crosslinkingly effective amount of N,N,N′,N′-tetraalkoxymethyl cyclohexanecarboguanamine crosslinking agent which is the product of Claim 1.

(iii) an acid cure catalyst.

9. The composition of Claim 8 wherein the polyfunctional material is an acrylic resin.

10. The composition of Claim 8 wherein the polyfunctional material is a polyester resin.